# EUROPEAN PATENT SPECIFICATION

(11) **EP 3 706 822 B1**
(45) Date of publication and mention of the grant of the patent: **30.09.2026**
(21) Application number: 18876845.1
(22) Date of filing: 08.11.2018
(51) Int. Cl.: A61L 31/04, A61M 25/00, A61M 39/02, A61L 29/04

(54) **SYSTEMS AND METHOD FOR MEDICAL DEVICE STRAIN RELIEF**
SYSTEME UND VERFAHREN ZUR ZUGENTLASTUNG EINES MEDIZINPRODUKTS
SYSTÈMES ET PROCÉDÉ DE SOULAGEMENT DE CONTRAINTE DE DISPOSITIF MÉDICAL

(30) Priority: 10.11.2017 US 201762584384 P
(43) Date of publication of application: 16.09.2020
(73) Proprietor: Merit Medical Systems, Inc., South Jordan, UT 84095 (US)
(72) Inventor: SEILER, Louis, Huntington Beach California 92649 (US); HALL, John, North Salt Lake Utah 84054 (US); RAINES, Westin, Eagle Mountain Utah 84005 (US)
(74) Representative: Cleveland Scott York
(86) International application number: PCT/US2018/059839
(87) International publication number: WO 2019/094599

(56) References cited:
- WO-A2-2007/061787
- CN-A- 102 753 106
- US-A- 5 549 109
- US-A1- 2002 072 712
- US-A1- 2011 060 264
- US-A1- 2013 060 268
- US-A1- 2013 202 721
- US-A1- 2015 196 734

## Description

### RELATED APPLICATIONS

This application claims priority to United States Provisional Application No. 62/584,384, filed on November 10, 2017 and tiled, "System and Method for Medical Device Strain Relief".

### TECHNICAL FIELD

The present disclosure relates generally to medical devices. More specifically, the present disclosure relates to strain relief elements which prevent or reduce kinking of medical device components. In some embodiments, the strain relief elements may facilitate laminar flow in a medical device lumen by minimizing kinking or sharp bends in the lumen. The present disclosure also describes methods of using a medical device with a strain relief component to prevent or reduce kinking of the medical device component when implanted into a patient. US 2011/ 060264 A1 describes a vascular access system that contemplates a catheter section adapted for insertion into a vein and a graft section adapted for attachment to an artery. WO 2007/061787A2 describes a vascular access system comprising graft material reinforced with expanded PTFE to resist stretching of the graft material and thereby resist leakage associated with stretched or bent graft material.

### BRIEF DESCRIPTION OF THE DRAWINGS

The embodiments disclosed herein will become more fully apparent from the following description and appended claims, taken in conjunction with the accompanying drawings. The drawings depict only typical embodiments, which embodiments will be described with additional specificity and detail in connection with the drawings in which:
FIG. 1 is a perspective view of a portion of a vascular access system.
FIG. 2 is an exploded perspective view of portions of the vascular access system of FIG. 1.
FIG. 3A is a side view of a portion of the vascular access system of FIG. 1.
FIG. 3B is a cross-section view of an inflow conduit and strain relief of the vascular access system of FIG. 1.
FIG. 3C is a longitudinal cross-section view of a portion of the inflow conduit and strain relief of the vascular access system of FIG. 1.
FIG. 4 is a side view of a portion of the inflow conduit and strain relief of the vascular access system of FIG. 1 with a portion of the strain relief removed from the inflow conduit.

### DETAILED DESCRIPTION

The scope of this invention is defined by the claims. Embodiments in the description relating to methods of treatment are not covered by the claims. Any "embodiment" or "example" which is disclosed in the description but is not covered by the claims should be considered as presented for illustrative purpose only.

Vascular access for performing hemodialysis for treatment of patients suffering from renal disease may be achieved by creating an arteriovenous (AV) anastomosis whereby a vein is attached to an artery to form a high-flow shunt or fistula. In some procedures, the AV fistula is accessed for hemodialysis with two large bore needles three times a week for a four-hour hemodialysis treatment session. As compared to some methods of hemodialysis vascular access, and AV fistula may provide longer viability, lower infection rates, and lower maintenance costs. However, an AV fistula may fail due to occlusion of the vein caused by thrombosis.

An alternative type of vascular access may be an implanted vascular access system that is connected to an artery at one end and is inserted into the central venous vasculature at a second end. Such a vascular access system may comprise a puncturable inflow conduit to be connected to an artery, an outflow conduit to be inserted into the central venous vasculature, and a connector connecting the two conduits. A vascular access system of this type may be connected to an artery in the upper arm and tunneled beneath the skin to the neck where the outflow conduit is inserted into the central venous vasculature. In this manner, a thrombosed vein may be bypassed. However, because a portion of the vascular access system is located in the upper arm of a patient, the conduit may tend to kink or be bent sharply when the arm is moved. A kinked or sharply bent conduit may result in non-laminar blood flow within the conduit leading to thrombosis and occlusion of the vascular access system.

A component configured to provide strain relief to such conduits may lessen instances of kinking or sharp bending.

Embodiments may be understood by reference to the drawings, wherein like parts are designated by like numerals throughout. It will be readily understood by one of ordinary skill in the art having the benefit of this disclosure that the components of the embodiments, as generally described and illustrated in the figures herein, could be arranged and designed in a wide variety of different configurations. Thus, the following more detailed description of various embodiments, as represented in the figures, is not intended to limit the scope of the disclosure, but is merely representative of various embodiments. While the various aspects of the embodiments are presented in drawings, the drawings are not necessarily drawn to scale unless specifically indicated.
It will be appreciated that various features are sometimes grouped together in a single embodiment, figure, or description thereof for the purpose of streamlining the disclosure. Many of these features may be used alone and/or in combination with one another.

The phrases "coupled to" and "in communication with" refer to any form of interaction between two or more entities, including mechanical, electrical, magnetic, electromagnetic, fluid, and thermal interaction. Two components may be coupled to or in communication with each other even though they are not in direct contact with each other. For example, two components may be coupled to or in communication with each other through an intermediate component.

The directional terms "distal" and "proximal" are given their ordinary meaning in the art in the context of the vascular system. That is, the distal end of a vascular access system refers to the end of the device farthest from the patient's heart. The proximal end refers to the opposite end, or the end nearest the patient's heart.

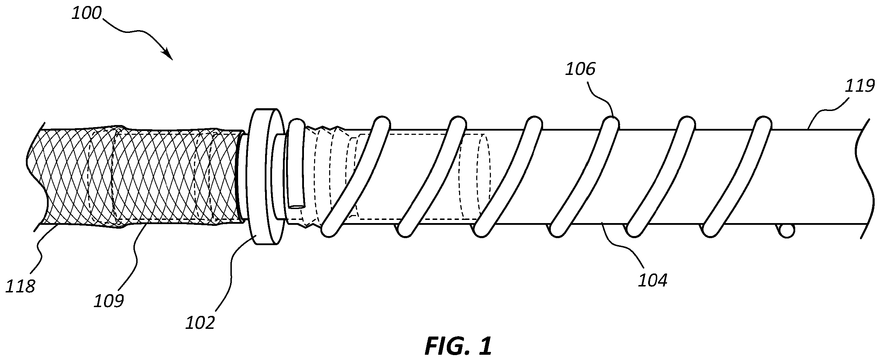

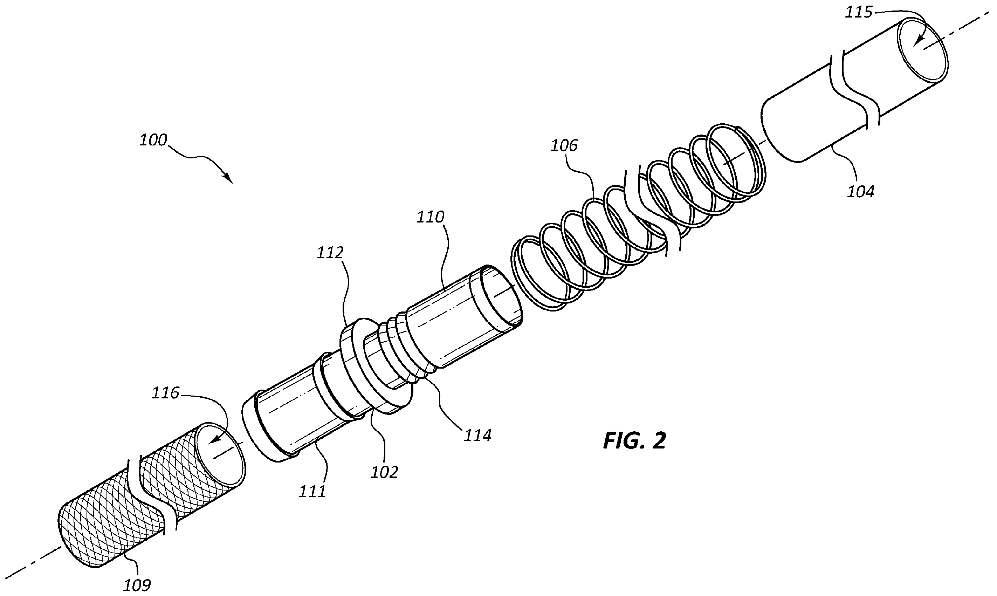

FIGS. 1-4 illustrate different views of a vascular access system and related components. In certain views each component may be coupled to, or shown with, additional components not included in every view. Further, in some views only selected components are illustrated, to provide detail into the relationship of the components. Some components may be shown in multiple views, but not discussed in connection with every view. Disclosure provided in connection with any figure is relevant and applicable to disclosure provided in connection with any figure or embodiment.

FIGS. 1- 4 depict an embodiment of a vascular access system 100. In the illustrated embodiment, the vascular access system 100 is comprised of an inflow conduit 104, an outflow conduit 109, a connector 102, and a strain relief 106. Referring to FIGS. 1-2, the connector 102 is configured to couple the inflow conduit 104 and the outflow conduit 109 together such that a single flow channel extends from one end of the vascular access system 100 to the opposite end. The direction of blood flow through the vascular access system 100 is from the distal end of the inflow conduit 104 which is anastomosed to the artery to the proximal end of the outflow conduit 109 which is inserted into the patient's central vasculature.

In some embodiments, the connector 102 may comprise an inflow arm 110, an outflow arm 111, a lumen 108, and a flange 112 disposed between the inflow arm 110 and the outflow arm 111. The inflow arm 110 may comprise one or more retention features 114, for example rings or barbs configured to engage with and retain the inflow conduit 104 when a proximal end of the inflow conduit 104 is slidingly coupled to the inflow arm 110. The outflow arm 111 may also comprise the one or more retention features 114 configured to engage with and retain the outflow conduit 109 when the distal end of the outflow conduit 109 is slidingly coupled to the outflow arm 111. In some embodiments, the inflow conduit 104 and the outflow conduit 109 may be coupled to the connector 102 using any suitable technique, such as adhesive, bands, clamps, etc.

Both the proximal end of the inflow conduit 104 and the distal end of the outflow conduit 109 may abut the flange 112 when coupled to the connector 102. The lumen 108 of the connector 102 may be configured to be equivalent in diameter to an inflow conduit lumen 115 and an outflow conduit lumen 116 such that there is a constant luminal surface to promote laminar blood flow from the inflow conduit lumen 115, through the connector lumen 108, and into the outflow conduit lumen 116 and thus prevent occlusion of the connector by thrombosed blood. The connector 102 may be formed from any suitable rigid, hemocompatible material, such as titanium, stainless steel, steel alloys, rigid plastics, etc.

The outflow conduit 109 comprises an elongate, tubular body 118 having the lumen 116. The outflow conduit 109 may be formed from any suitable flexible, hemocompatible material, such as polyurethane, silicone rubber, copolymers of polyurethane and silicone, etc. The outflow conduit 109 may be reinforced with wire or thread braiding such that the outflow conduit 109 is resistant to kinking and crushing. The outflow conduit 109 may be configured to be trimmable such that the length of the outflow conduit 109 can be customized by a healthcare worker to fit the patient.

The inflow conduit 104 comprises an elongate, tubular body 119 having the lumen 115. The inflow conduit 104 is formed from any suitable hemocompatible, puncturable material, such as polytetrafluoropropylene (PFTE), polyurethane, etc. The inflow conduit 104 is configured to be punctured with multiple large bore arteriovenous fistula needles without significant damage to the inflow conduit 104 causing leakage and/or thrombus formation. The inflow conduit 104 may be configured to be trimmable such that the length of the inflow conduit 104 can be customized by the healthcare worker to fit the patient. In some embodiments, the distal end of the inflow conduit 104 is configured to be trimmable such that the distal end can be anastomosed to the artery of the patient.

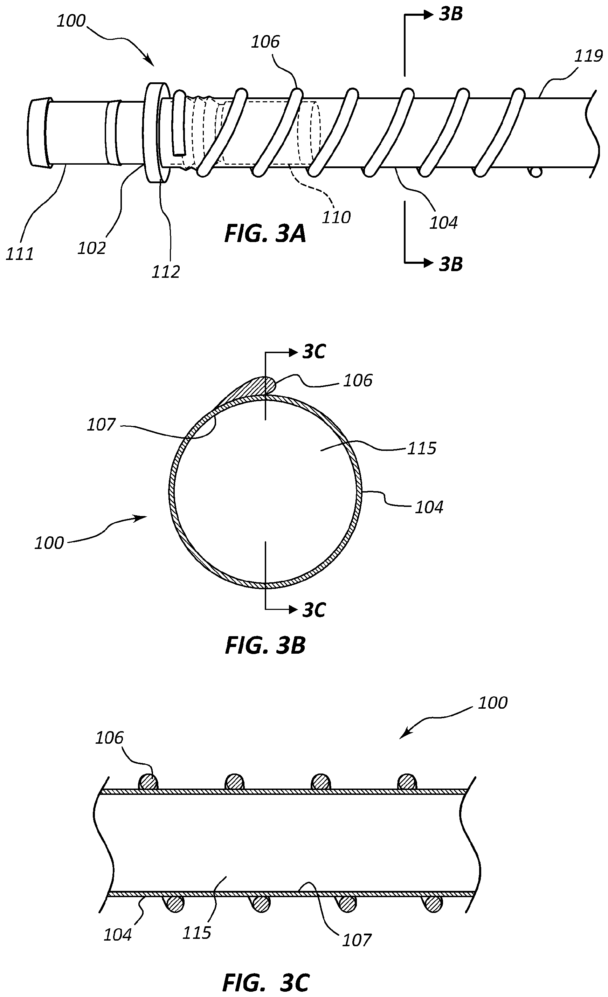

Referring to FIGS. 3A-3C, the connector 102, the inflow conduit 104, and the strain relief 106 of the vascular access system 100 are illustrated. The inflow conduit 104 is shown to comprise the strain relief 106 coupled to the outside surface of the inflow conduit 104. The strain relief 106 is configured to prevent kinking of the inflow conduit 104, particularly in the area where the inflow conduit 104 couples to the connector 102. The strain relief 106 is configured to maintain a circular shape of the inflow conduit 104 when the inflow conduit 104 is bent sharply at the end of the inflow arm 110 of the connector 102 or at other portions of the inflow conduit 104 when the vascular access system 100 is implanted in a patient. The strain relief 106 is disposed around and coupled to an outer surface of the inflow conduit 104. A proximal end of the strain relief 106 is disposed adjacent to the proximal end of the inflow conduit 104. The strain relief 106 may extend longitudinally along the inflow conduit 104 toward the distal end of the inflow conduit 104. In some embodiments, the length of the strain relief 106 may extend from the proximal end to the distal end of the inflow conduit 104. In another embodiment, the strain relief 106 length may be along only a portion of the inflow conduit 104 such that the strain relief may extend from 2 cm to 100 cm, including 3 cm to 20 cm from the proximal end of the inflow conduit 104. In certain embodiments the strain relief 106 may be segmented such that the strain relief 106 is coupled to portions of the inflow conduit 104 with gaps between the segments of the strain relief 106. The strain relief 106 may be tapered from a proximal end to a distal end such that the distal end has a larger diameter than the proximal end.

In some embodiments, the strain relief 106 may be configured to have an inner diameter approximately equivalent to the outer diameter of the inflow conduit 104 such that the strain relief 106 does not reduce a diameter of or restrict the lumen 115 of the inflow conduit 104 resulting in a disrupted or uneven inside surface 107 in an area where the strain relief 106 is coupled to the inflow conduit 104. Continuity along the inside surface 107 of the inflow conduit 104 may promote laminar flow of blood and prevent thrombosis of the inflow conduit 104.

The strain relief 106 may comprise a wire or polymer elongate coil. I According to the claimed invention, the strain relief 106 comprises a copolymer. The copolymer comprises monomers of hexafluoropropylene and tetrafluoroethylene, whereby the chemical structure of the copolymer is 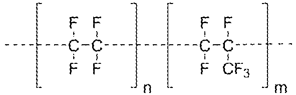 where "n" is an integer of 1 or greater and "m" is an integer of 1 or greater. In some embodiments the strain relief 106 comprises fluorinated ethylene propylene (FEP). In other embodiments the strain relief 106 comprises polytetrafluoroethylene (PTFE), having a chemical structure of 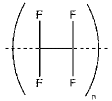 where "n" is an integer of 1 or greater. Other polymers and copolymers are contemplated. The strain relief 106 may comprise some segments which comprise various copolymers or polymers. In some embodiments the strain relief 106 may comprise segments of FEP in addition to other copolymers or polymers. For example, the strain relief 106 may comprise FEP and PTFE, FEP and perfluoroalkoxy poly resin, or polypropylene. The strain relief 106 may comprise a suitable radio-opaque filler, such as titanium dioxide, barium sulfate, bismuth subcarbonate, bismuth oxychloride, tungsten, etc., such that the strain relief 106 is visible under X-ray radiation. In some embodiments the strain relief 106 comprises other fillers, such as carbon, pigment, etc., such that the strain relief 106 can be easily visualized by the healthcare worker against the inflow conduit 104.

As shown in FIGS. 3A-3C, the strain relief 106 is configured in the shape of an elongate coil. The strain relief 106 may be formed into the elongate, coil shape utilizing any suitable manufacturing technique. In some embodiments the strain relief 106 is heat set into the elongate, coil form. For example, the strain relief 106 may be extruded as a filament or rod which is then wound around a mandrel and heat set to create an elongate, coil shape. The diameter of the filament or rod may range from 0.254 mm to 2.540 mm, including 0.508 mm to 1.524 mm. The inner diameter of the strain relief 106 may range from approximately 2 mm to approximately 12 mm, including from approximately 4 mm to approximately 10 mm. In some embodiments the rod or filament of the strain relief 106 is extruded into the elongate, coil shape. The strain relief 106 may be etched or cut from a polymer sheet. In certain embodiments the rod or filament of the strain relief 106 is stretched after it is manufactured and before it is formed into the elongate, coil shape. The strain relief 106 may be configured such that a gap between each coil ranges from 0.00 mm to 25.40 mm, including from 2.54 mm to 6.35 mm.

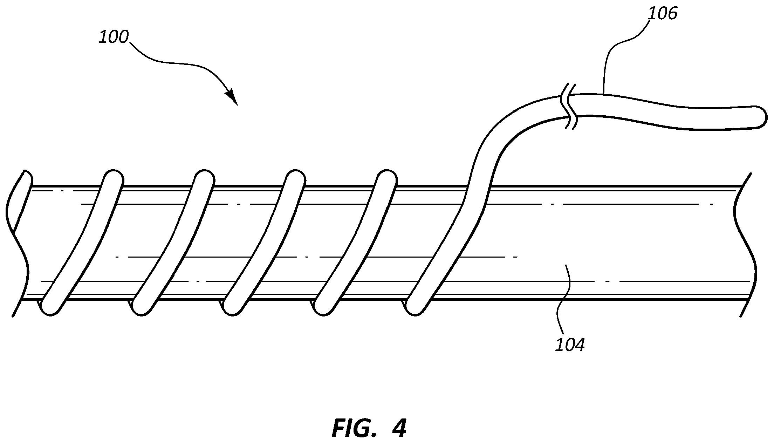

Referring to FIG. 4, the strain relief 106 is configured to be releasably coupled to the inflow conduit 104 such that the strain relief 106 may be peeled from the inflow conduit 104 by a healthcare worker to adjust the length of the strain relief 106 coupled to the inflow conduit 104. An end of the strain relief 106 may be grasped by the fingers of the healthcare worker or by a medical instrument and lifted from the inflow conduit 104. A desired length of the strain relief 106 may be peeled from the inflow conduit 104. Alternatively, a portion of the strain relief 106 between the proximal and distal ends may be removed from the inflow conduit 104. The length of the strain relief 106 removed from the inflow conduit 104 may be trimmed with a sharp medical instrument.

The strain relief 106 is coupled to the outside surface of the inflow conduit 104 utilizing heat, whereby preferably the strain relief 106 is heated to a temperature approximately equivalent to the melting point of the material of the strain relief 106 but less than a melting temperature of the material of the inflow conduit 104 such that the strain relief 106 releasably couples to the surface of the inflow conduit 104 and the two materials do not meld together. The strain relief 106 may be heated for a time period of between about 30 seconds and five minutes, including one minute to two minutes.

According to the claimed invention, different sections of the strain relief 106 are heated at different temperatures and for different lengths of time such that the strain relief 106 is coupled at various strengths so that some sections of the strain relief 106 are more strongly coupled than others. The exterior surface of the strain relief 106 and/or the inflow conduit 104 may be modified using any suitable process, such as chemical etching, electro etching, vapor deposition, plasma, etc., to customize the coupling force of the strain relief 106 to the surface of the inflow conduit 104.

In some embodiments the strain relief 106 is configured to minimize or eliminate bulging of the internal diameter of the inflow conduit 104 after the strain relief 106 is adhered to the inflow conduit 104. The strain relief 106 is configured to maintain laminar flow of blood through the inflow conduit 104 in the area where the strain relief 106 is coupled to the inflow conduit 104. The inside surface 107 of the lumen 115 of the inflow conduit 104 is configured to be unchanged in the region where the strain relief 106 is coupled such that grooves or other surface irregularities are not formed on the inside surface 107 of the lumen 115.

In use (not part of the claimed invention), the vascular access system 100 comprising the outflow conduit 109, the connector 102, the inflow conduit 104, and the strain relief 106 releasably coupled to the inflow conduit 104 is implanted by the healthcare worker into the patient requiring vascular access for hemodialysis treatments. The proximal end of the outflow conduit 109 is inserted into a vessel of the central venous system and the elongate body 118 is tunneled beneath the patient's skin to an exit site in the upper arm. The distal end of the inflow conduit 104 is tunneled from the exit site to a site near the elbow and anastomosed to an artery in the patient's arm. The proximal end of the inflow conduit 104 and the distal end of the outflow conduit 109 are coupled to the connector 102 such that a fluid tight seal is created.

The strain relief 106 is configured to extend proximally from adjacent to the flange 112 of the connector 102 to beyond the end of the inflow arm 110 such that kinking of the inflow conduit 104 at the end of the inflow arm 110 is prevented. Excess length of the strain relief 106, as determined by the healthcare worker, may be peeled from the inflow conduit 104. Alternatively, portions of the strain relief 106 in areas of the inflow conduit 104 that are not at risk of kinking may be selectively removed by the healthcare worker. The strain relief 106 can be removed or peeled from the inflow conduit 104 when the healthcare worker grasps a portion of the strain relief 106 with fingers or a medical instrument and applies a lifting force to the portion of the strain relief 106. When the desired length of the strain relief 106 has been removed, the healthcare worker can trim the strain relief 106 with a sharp medical instrument, such as scissors, surgical blade, etc., and discard the excess portion of the strain relief 106.

Any methods disclosed herein include one or more steps or actions for performing the described method. The method steps and/or actions may be interchanged with one another. In other words, unless a specific order of steps or actions is required for proper operation of the embodiment, the order and/or use of specific steps and/or actions may be modified. Moreover, sub-routines or only a portion of a method described herein may be a separate method within the scope of this disclosure. Stated otherwise, some methods may include only a portion of the steps described in a more detailed method.

## Claims

1. A vascular access system (100) comprising:
an inflow conduit (104); and
a connector (102);
a strain relief component (106) comprising a copolymer having a chemical structure of 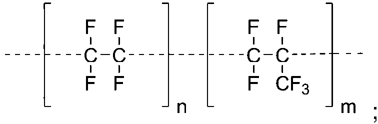 where "n" is an integer of 1 or greater and "m" is an integer of 1 or greater,
wherein the strain relief component (106) is releasably coupled to the inflow conduit (104), and
wherein the strain relief component (106) comprises a plurality of sections and different sections of the strain relief component (106) are heated at different temperatures and for different lengths of time such that the strain relief component (106) is coupled at various strengths so that some sections of the strain relief component (106) are coupled to the inflow conduit (104) more strongly than others.

2. The vascular access system (100) of claim 1, wherein the strain relief component (106) is configured to be, at least in part, peeled from the inflow conduit (104).

3. The vascular access system (100) of any one of claim 1 or claim 2, wherein the strain relief component (106) resists kinking of the inflow conduit (104).

4. The vascular access system (100) of any one of claims 1 to 3, wherein the strain relief component (106) is hemocompatible.

5. The vascular access system (100) of any one of claims 1 to 4, wherein the strain relief component (106) is an elongate coil.

6. The vascular access system (100) of any one of claims 1 to 5, wherein the strain relief component (106) has a circular shaped cross-section.

7. The vascular access system (100) of any one of claims 1 to 6, wherein a lumen (115) of the inflow conduit (104) has a smooth surface where the strain relief component (106) is attached to the inflow conduit (104).

8. The vascular access system (100) of any one of claims 1 to 7, wherein blood maintains laminar flow through the inflow conduit lumen (115) where the strain relief component (106) is attached to the inflow conduit (104).

9. The vascular access system (100) of any one of claims 1 to 8, wherein the strain relief component (106) comprises polypropylene.

10. The vascular access system (100) of claim 1, wherein the strain relief component (106) comprises fluorinated ethylene propylene (FEP).

11. The vascular access system (100) of claim 1, wherein the strain relief component (106) comprises polytetrafluorethylene (PTFE).

12. The vascular access system (100) of claim 3, wherein the peeled part of the strain relief component (106) is configured to be trimmed.

13. A method of forming a vascular access system (100) resistant to kinking, comprising:
providing an inflow conduit (104);
providing a connector (102);
providing the vascular access system (100) of claim 1; and
coupling the strain relief component (106) to the inflow conduit (104) by heating different sections of the strain relief component (106) at different temperatures and for different lengths of time such that the strain relief component (106) is coupled to the inflow conduit (104) at various strengths.

14. The method of claim 13, wherein coupling the strain relief component (106) to the inflow conduit (104) comprises:
disposing the strain relief component (106) around the inflow conduit (104); and
heating the strain relief component (106) and inflow conduit (104) to a melt temperature of the copolymer of the strain relief component (106) but below a melt temperature of a material of the inflow conduit (104).

## Patentansprüche

1. Vaskuläres Zugangssystem (100), das Folgendes umfasst:
eine Zuflussleitung (104); und
ein Verbindungselement (102);
eine Zugentlastungskomponente (106), die ein Copolymer umfasst, das eine chemische Struktur von 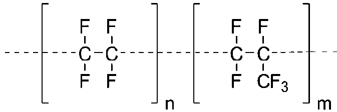 aufweist; worin "n" eine ganze Zahl von 1 oder größer ist und "m" eine ganze Zahl von 1 oder größer ist,
wobei die Zugentlastungskomponente (106) lösbar mit der Zuflussleitung (104) verbunden ist und
wobei die Zugentlastungskomponente (106) eine Vielzahl von Abschnitten umfasst und unterschiedliche Abschnitte der Zugentlastungskomponente (106) bei unterschiedlichen Temperaturen und für unterschiedliche Zeitspannen erhitzt werden, sodass die Zugentlastungskomponente (106) bei verschiedenen Stärken verbunden ist, sodass einige Abschnitte der Zugentlastungskomponente (106) stärker mit der Zuflussleitung (104) verbunden sind als andere.

2. Vaskuläres Zugangssystem (100) nach Anspruch 1, wobei die Zugentlastungskomponente (106) konfiguriert ist, um zumindest teilweise von der Zuflussleitung (104) abgezogen zu werden.

3. Vaskuläres Zugangssystem (100) nach einem von Anspruch 1 oder Anspruch 2, wobei die Zugentlastungskomponente (106) dem Abknicken der Zuflussleitung (104) widersteht.

4. Vaskuläres Zugangssystem (100) nach einem der Ansprüche 1 bis 3, wobei die Zugentlastungskomponente (106) hämokompatibel ist.

5. Vaskuläres Zugangssystem (100) nach einem der Ansprüche 1 bis 4, wobei die Zugentlastungskomponente (106) eine langgestreckte Spirale ist.

6. Vaskuläres Zugangssystem (100) nach einem der Ansprüche 1 bis 5, wobei die Zugentlastungskomponente (106) einen kreisförmigen Querschnitt aufweist.

7. Vaskuläres Zugangssystem (100) nach einem der Ansprüche 1 bis 6, wobei ein Lumen (115) der Zuflussleitung (104) eine glatte Oberfläche aufweist, wo die Zugentlastungskomponente (106) an der Zuflussleitung (104) angebracht ist.

8. Vaskuläres Zugangssystem (100) nach einem der Ansprüche 1 bis 7, wobei Blut einen laminaren Fluss durch das Zuflussleitungslumen (115) beibehält, wo die Zugentlastungskomponente (106) an der Zuflussleitung (104) angebracht ist.

9. Vaskuläres Zugangssystem (100) nach einem der Ansprüche 1 bis 8, wobei die Zugentlastungskomponente (106) Polypropylen umfasst.

10. Vaskuläres Zugangssystem (100) nach Anspruch 1, wobei die Zugentlastungskomponente (106) fluoriertes Ethylenpropylen (FEP) umfasst.

11. Vaskuläres Zugangssystem (100) nach Anspruch 1, wobei die Zugentlastungskomponente (106) Polytetrafluorethylen (PTFE) umfasst.

12. Vaskuläres Zugangssystem (100) nach Anspruch 3, wobei der abgezogene Teil der Zugentlastungskomponente (106) zum Beschneiden konfiguriert ist.

13. Verfahren zur Bildung eines vaskulären Zugangssystems (100), das dem Abknicken widersteht, das Folgendes umfasst:
Bereitstellen einer Zuflussleitung (104);
Bereitstellen eines Verbindungselements (102);
Bereitstellen der Zugentlastungskomponente (106) nach Anspruch 1; und
Verbinden der Zugentlastungskomponente (106) mit der Zuflussleitung (104) durch Erhitzen von unterschiedlichen Abschnitten der Zugentlastungskomponente (106) bei unterschiedlichen Temperaturen und für unterschiedliche Zeitspannen, sodass die Zugentlastungskomponente (106) bei verschiedenen Stärken mit der Zuflussleitung (104) verbunden wird.

14. Verfahren nach Anspruch 13, wobei das Verbinden der Zugentlastungskomponente (106) mit der Zuflussleitung (104) Folgendes umfasst:
Anordnen der Zugentlastungskomponente (106) um die Zuflussleitung (104) herum; und
Erhitzen der Zugentlastungskomponente (106) und Zuflussleitung (104) auf eine Schmelztemperatur des Copolymers der Zugentlastungskomponente (106), doch unterhalb einer Schmelztemperatur eines Materials der Zuflussleitung (104).

## Revendications

1. Système d'accès vasculaire (100) comprenant :
un conduit d'alimentation (104) ; et
un connecteur (102) ;
un élément réducteur de tension (106) comprenant un copolymère répondant à une structure chimique 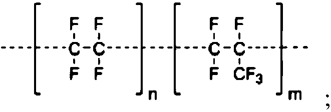 où « n » est un nombre entier égal ou supérieur à 1 et « m » est un nombre entier égal ou supérieur à 1,
dans lequel l'élément réducteur de tension (106) est couplé de manière amovible au conduit d'alimentation (104), et
où l'élément réducteur de tension (106) comprend une pluralité de sections et différentes sections de l'élément réducteur de tension (106) sont chauffées à des températures différentes et pendant des durées différentes de telle sorte que l'élément réducteur de tension (106) est couplé selon des forces variables, de sorte que certaines sections de l'élément réducteur de tension (106) sont couplées au conduit d'alimentation (104) plus fortement que d'autres.

2. Système d'accès vasculaire (100) selon la revendication 1, dans lequel l'élément réducteur de tension (106) est configuré pour être, au moins en partie, décollé du conduit d'alimentation (104).

3. Système d'accès vasculaire (100) selon l'une quelconque parmi la revendication 1 ou la revendication 2, dans lequel l'élément réducteur de tension (106) empêche le conduit d'alimentation (104) de subir des pliures.

4. Système d'accès vasculaire (100) selon l'une quelconque des revendications 1 à 3, dans lequel l'élément réducteur de tension (106) est hémocompatible.

5. Système d'accès vasculaire (100) selon l'une quelconque des revendications 1 à 4, dans lequel l'élément réducteur de tension (106) est une spirale allongée.

6. Système d'accès vasculaire (100) selon l'une quelconque des revendications 1 à 5, dans lequel l'élément réducteur de tension (106) présente une section transversale de forme circulaire.

7. Système d'accès vasculaire (100) selon l'une quelconque des revendications 1 à 6, dans lequel une lumière (115) du conduit d'alimentation (104) présente une surface lisse à l'endroit où l'élément réducteur de tension (106) est fixé au conduit d'alimentation (104).

8. Système d'accès vasculaire (100) selon l'une quelconque des revendications 1 à 7, dans lequel le sang conserve un écoulement laminaire à travers la lumière (115) du conduit d'alimentation à l'endroit où l'élément réducteur de tension (106) est fixé au conduit d'alimentation (104).

9. Système d'accès vasculaire (100) selon l'une quelconque des revendications 1 à 8, dans lequel l'élément réducteur de tension (106) comprend du polypropylène.

10. Système d'accès vasculaire (100) selon la revendication 1, dans lequel l'élément réducteur de tension (106) comprend de l'éthylène-propylène fluoré (FEP).

11. Système d'accès vasculaire (100) selon la revendication 1, dans lequel l'élément réducteur de tension (106) comprend du polytétrafluoroéthylène (PTFE).

12. Système d'accès vasculaire (100) selon la revendication 3, dans lequel la partie décollée de l'élément réducteur de tension (106) est configurée pour être rognée.

13. Méthode de formation d'un système d'accès vasculaire (100) résistant aux pliures, comprenant :
la mise à disposition d'un conduit d'alimentation (104) ;
la mise à disposition d'un connecteur (102) ;
la mise à disposition de l'élément réducteur de tension (106) selon la revendication 1 ; et
le couplage de l'élément réducteur de tension (106) au conduit d'alimentation (104) en chauffant différentes sections de l'élément réducteur de tension (106) à des températures différentes et pendant des durées différentes de telle sorte que l'élément réducteur de tension (106) est couplé au conduit d'alimentation (104) selon des forces variables.

14. Méthode selon la revendication 13, dans laquelle le couplage de l'élément réducteur de tension (106) au conduit d'alimentation (104) comprend :
la mise en place de l'élément réducteur de tension (106) autour du conduit d'alimentation (104) ; et
le chauffage de l'élément réducteur de tension (106) et du conduit d'alimentation (104) à une température de fusion du copolymère de l'élément réducteur de tension (106), mais en dessous d'une température de fusion d'un matériau du conduit d'alimentation (104).
